(19) Europäisches Patentamt
European Patent Office
Office européen des brevets

(11) Veröffentlichungsnummer: **0 000 900**
**B1**

(12) # EUROPÄISCHE PATENTSCHRIFT

(21) Anmeldenummer: **78100629.1**

(22) Anmeldetag: **09.08.78**

(51) Int. Cl.³: **C 07 D 493/10,**
**B 41 M 5/16**

(54) **Spirodipyrane, deren Verwendung und diese enthaltende druckempfindliche Aufzeichnungsmaterialien**

(30) Priorität: **18.08.77 DE 2737207**

(43) Veröffentlichungstag der Anmeldung:
**07.03.79 Patentblatt 79/05**

(45) Bekanntmachung des Hinweises auf die Patenterteilung:
**14.01.81 Patentblatt 81/02**

(84) Benannte Vertragsstaaten:
**CH DE FR GB**

(56) Entgegenhaltungen:
**DE - A - 2 019 822**
**DE - A - 2 232 364**
**DE - A - 2 323 803**
**DE - A - 2 430 568**
**DE - A - 2 611 600**
**GB - A - 889 586**
**US - A - 3 022 318**

(73) Patentinhaber: **BASF Aktiengesellschaft**
**Carl-Bosch-Strasse 38**
**D - 6700 Ludwigshafen (DE)**

(72) Erfinder: **Baumann, Hans, Dr.**
**Roemerweg 31**
**D - 6706 Wachenheim (DE)**
**Oberlinner, Andreas, Dr.**
**Bruesseler Ring 53**
**D - 6700 Ludwigshafen (DE)**

**EP 0 000 900 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Spirodipyrane, deren Verwendung und diese enthaltende druckempfindliche Aufzeichnungsmaterialien

Die Erfindung betrifft Spirodipyrane der allgemeinen Formel

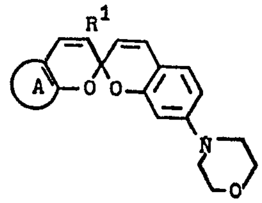

(I),

in der

A den Rest eines ankondensierten Benzolringes oder einen in 2,1-Stellung ankondensierten Naphthalinring und

$R^1$ $C_1$- bis $C_{16}$-Alkyl bedeutet.

Die Spirodipyrane der Formel I sind schwach farbige bis farblose Verbindungen, deren Lösungen in einem inerten, organischen Lösungsmittel in Kontakt mit elektroenanziehenden Substanzen rotviolette bis blaue Färbungen geben. Typische Beispiele für Elektronenakzeptorsubstanzen sind Carbon- oder Mineralsäuren, Kaolin, Bentonit, aktivierter Ton, Aluminiumsilikat, Attapulgit oder jeder beliebige Ton, sauer reagierende polymere Materialien wie Kondensationsprodukte aus Phenolen und/oder Phenolsulfonsäuren, ferner Metalloxide oder -salze wie Zinkoxid, Aluminiumoxid, Zinkchlorid, Eisenstearat oder Cobaltnaphthenat.

Aufgrunde dieser Eigenschaften sind die neuen Spirodipyrane der Formel I als Farbbildner für druckempfindliche Aufzeichnungsmaterialen oder Kopiermaterialien geeignet.

Die erfindungsgemäßen Spirodipyrane der Formel I haben in druckempfindlichen Durchschreibesystemen den Vorteil, daß die Farbbildner auf nicht beschichtetem Streichrohpapier praktisch keine Tendenz zur Farbbildung zeigen. Beim Durchschreiben entsteht daher auf der Rückseite des mit dem Farbbildner beschichteten Deckblattes keine Spiegelschrift. Aus dem gleichen Grund erfolgt bei ungewollter Zerstörung der Kapseln keine Verschmutzung (Anfärbung) der Seite des Blattes, welche die Mikrokapselschicht trägt.

Die erfindungsgemäßen Spirodipyrane haben gegenüber dem aus den DE—OS 22 32 364, 23 23 803 und 24 30 568 bekannten Spirodipyranen, die in der 7-Stellung eine Dialkylaminogruppe tragen, den Vorteil, daß die Spirodipyrane der vorliegenden Erfindung auf Streichrohpapier und auf nicht mit aktivem Clay beschichteten Papier praktisch keine Färbung geben. Z.B. färbt der aus Beispiel 1 der DE—OS 22 32 364 bekannte Farbbildner Streichrohpapier ungefähr 12,5mal so stark an wie der Farbstoff des Beispiels 8 der vorliegenden Erfindung. Im Vergleich zu diesem Farbbildner färben die aus der DE—OS 23 23 803, Beispiel 2 und aus der DE—OS 24 30 568, Beispiel 5 bekannten Farbbildner Streichrohpapier ungefähr 4mal, bzw. 3,2mal stärker an.

Gegenüber dem aus Beispiel 15 der DE—OS 26 11 600 bekannten Spirodipyran, das in der 7-Stellung eine Morpholingruppe und in der 7'-Stellung eine Diäthylaminogruppe trägt und das zu den Spirodipyranen der vorliegenden Erfindung als das nächstliegende anzusehen ist, weisen die Spirodipyrane der vorliegenden Erfindung bei gleichem Verhalten auf Streichrohpapier une wesentlich höhere Farbstärke auf. So beträgt die Farbstärke des aus Beispiel 15 der DE—OS 26 11 600 bekannten Farbbildners nur 29% der des Farbbildners aus Beispiel 2 der vorliegenden Erfindung.

Vorteilhaft ist es, die erfindungsgemäßen Spirodipyrane in bekannter Weise in organischen Lösungsmitteln wie Chlorparaffinen, halogeniertem oder teilhydriertem Biphenyl, Alkylbenzol, Alkylnaphthalin, alkyliertem Dibenzylbenzol, Paraffinöl, Mineralöl oder auch in üblichen Lösungsmitteln wie Toluol, Xylol, in Form einer Lösung oder Suspension in Mikrokapseln einzuschließen und damit unter Mitverwendung von Bindemitteln und weiteren Hilfsmitteln wie Abstandshaltern die Papieroberfläche zu beschichten. Man erhält druckempfindliche Papiere, die in kontakt mit elektronenanziehenden Materialien bei entsprechendem Schrieb- oder Typendruck ein Schriftbild in rotvioletter bis blauer Farbe geben.

Geeignete Verfahren zur Herstellung von Mikrokapseln sind z.B. in den US—PS 2 800 457 und 2 800 458 und in der DE—PS 21 19 933 beschrieben.

Da die erfindungsgemäßen Spirodipyrane (I) auch in wäßriger Suspension stabiler sind als Farbbildner, die Dialkylaminogruppen enthalten (DE—OS 22 32 364, 23 23 803 und 24 30 568), erhält man mit ersteren praktisch farblose Milrokapseldispersionen.

Man kann die erfindungsgemäßen Spirodipyrane der allgemeinen Formel I auch nach dem in der US—PS 3 103 404 beschriebenen Verfahren in Wachs oder Öl-Wachsmischungen fein verteilen und mit diesen Mischungen Träger, wie Folien oder Papier beschichten. Man erhält druckempfindliche Materialien, die zum Durchschreiben auf mit Elektronenakzeptorsubstanzen beschichteten Papieren geeignet sind und die nach Gebrauch wie Kohlepapier entfernt werden.

Für $R^1$ sind als $C_1$- bis $C_{16}$-Alkyl z.B. zu nennen: Methyl, Äthyl, n-Propyl, Isopropyl, n-Butyl, sec.

Butyl, Isobutyl, tert. Butyl, n-Pentyl, Isopentyl, n-Hexyl, n-Nonyl, i-Nonyl, n-Octyl, i-Octyl, n-Decyl, i-Decyl, n-Dodecyl, i-Dodecyl, n-Hexadecyl.

Aus technischen Gründen sind Verbindungen der Formel I bevorzugt, in der $R^1$ für $C_1$- bis $C_4$-Alkyl steht.

Besonders bevorzugte Farbbildner sind:

(Ib) 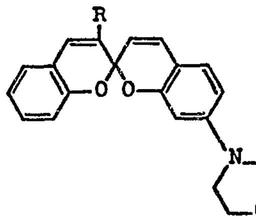 und 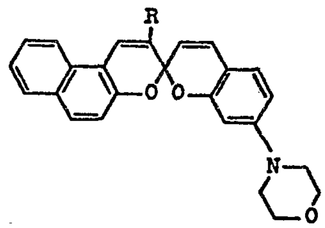 (Ic),

in denen R = $—CH_3$, $—C_2H_5$, $—CH(CH_3)_2$ oder $—CH_2—CH(CH_3)_2$ bedeuten.

Die Synthese der Farbbildner erfolgt nach dem folgenden Reaktionsschema in an sich bekannter Weise durch Cyclisierung der o-Hydroxylaryl-styryl-verbindungen der Formel IV. Letztere werden z.B. durch Kondensation von Benzopyryliumsalzen der Formel II mit N-substituierten p-Aminosalicylaldehyden der Formel III erhalten:

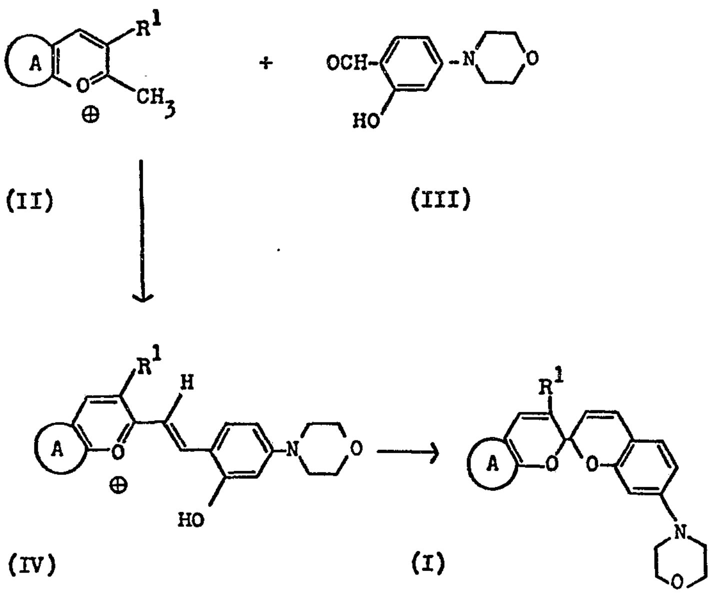

(II) (III) (IV) (I)

Die Kondensation erfolgt zweckmäßigerweise in inerten organischen Lösungsmitteln, wie Alkoholen, Carbonsäuren, Carbonsäureanhydriden, Carbonsäureamiden, Kohlenwasserstoffen oder Acetonitril, gegebenenfalls in Gegenwart saurer oder basischer Kondensationsmittel, wie Zinkchlorid, Phosphorsäure, Chlorwasserstoff, Toluolsulfonsäure, Borsäure, Pyridin, Piperidin, Triäthylamin, Ammoniumacetat unter üblichen Kondensationsbedingungen.

In der Regel wird die Kondensation bei Temperaturen im Bereich von 20 bis 120°C ausgeführt.

Der Ringschluß zum Pyranderivat kann zusammen mit der Kondensation oder anschließend an diese im selben oder in einem getrennten Arbeitsgang erfolgen. Der Ringschluß wird in Gegenwart von Basen, wie Natrium- oder Kaliumhydroxid, Natrium- oder Kaliumcarbonat, Natrium- oder Kaliumacetat, Ammoniak, aliphatischen Amine, Pyridin, in üblicher Weise durchgeführt. Die aus dieser Lösung sich abscheidenden kristallisierten Spirodipyranverbindungen sind direkt oder nach einer Reinigung z.B. durch Umkristallisieren oder Umfällen als Farbbildner für Kopierverfahren verwendbar.

Für die Herstellung der Verbindungen (IV) kommen als Ausgangsverbindungen der Formeln II und III z.B. in Betracht:

a) Pyryliumsalze der Formel II in Form ihrer Chloride, Perchlorate, Tetrafluorborate, Tetrachloroferrate, Trichlorzinkate:

2,3-Dimethyl-benzopyryliumsalz,
2-Methyl-3-i-propyl-benzopyryliumsalz,
2-Methyl-3-i-butyl-benzopyryliumsalz,
2-Methyl-3-decyl-benzopyryliumsalz,
2-Methyl-3-i-pentyl-benzopyryliumsalz,
2-Methyl-3-n-pentyl-benzopyryliumsalz,

2-Methyl-3-äthyl-benzopyryliumsalz,
2-Methyl-3-nonyl-benzopyryliumsalz,
2,3-Dimethyl-naphtho-(2,1-b)pyryliumsalz,
2-Methyl-3-i-propyl-naphtho-(2,1-b)pyryliumsalz,
2-Methyl-3-i-butyl-naphtho(2,1-b)pyryliumsalz,
2-Methyl-3-i-pentyl-naphtho(2,1-b)pyryliumsalz, und
b) als Aldehyd der Formel III: 4-N-Morpholinyl-salicylaldehyd.

Die Herstellung und Isolierung der Verbindungen der Formel I wird in den folgenden Ausführungsbeispielen weiter erläutert. Die im folgenden genannten Teile beziehen sich auf das Gewicht.

Beispiel 1

165 Teile 2,3-Dimethyl-benzopyrylium-trichlorozinkat und 105 Teile 4-N-Morpholinylsalicylaldehyd werden in 900 Teilen Methanol zwei Stunden unter Rückfluß erhitzt. Der kristalline Farbstoff wird aus dem abgekühlten Reaktionsgemisch isoliert und in 500 Teilen 25%iger Ammoniaklösung und 1 000 Teilen Toluol bis zur vollständigen Aufhellung gerührt. Die Toluolphase wird abgetrennt, mit Natriumsulfat getrocknet und auf ein Drittel ihres ursprünglichen Volumens eingeengt. Durch Zugabe von 250 Teilen Methanol werden aus dieser Lösung 130 Teilen 3'-methyl-7-N-morpholinyl-2,2'-spirodi-(2H-1-benzopyran) der Formel

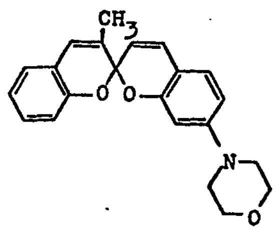

gefällt.
Schmelzpunkt 136 bis 138°C.

Wird eine Lösung dieser Verbindung in Mikrokapseln eingeschlossen und auf Papier als Beschichtung aufgebracht, so erhält man beim Auflegen und Beschriften auf einer sauren Nehmerschicht, wobei die Kapseln zerstört werden und deren Inhalt mit der Nehmerschicht in Berührung gebracht wird, eine intensive blaue Durchschrift.

Aufgrund der sehr geringen Selbstfarbentwicklungsfähigkeit des Farbbildners erfolgt praktisch keine Farbenentwicklung (Spiegelschrift) auf dem die Kapselschicht tragenden Blatt durch die aus den zerstörten Kapseln freigesetzte Farbbildnerlösung.

Diese geringe Farbbildungstendenz zeigt der Farbbildner auch beim Durchschreiben auf nicht beschichtetem Papier, wo nahezu keine Farbentwicklung erfolgt, während ein Farbbildner mit einer Diäthylaminogruppe anstelle des Morpholinringes eine deutlich sichtbare, blaue Durchschrift entwickelt.

Beispiele 2 bis 7

Analog den Angaben in Beispiel 1 werden Farbbildner der Formel

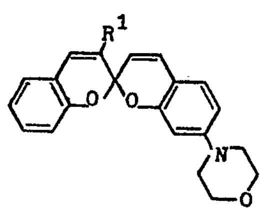

hergestellt.

| Nr. | $R^1$ | Farbton |
|---|---|---|
| 2 | $CH(CH_3)_2$ | blau |
| 3 | $C_{16}H_{33}$ | blau |
| 4 | $C_5H_{11}(n)$ | blau |
| 5 | $C_{10}H_{11}(n)$ | blau |
| 6 | $C_{16}H_{33}$ | blau |
| 7 | $—C_2H_5$. | blau |

Beispiel 8

19 Teile 2,3-Dimethyl-naphthopyryliumtrichlorozinkat und 11 Teile 4-N-Morpholinylsalicylaldehyd werden in 150 Teilen Alkohol zwei Stunden unter Rückfluß erhitzt. Der kristalline Farbstoff wird isoliert und wie in Beispiel 1 beschrieben in die farblose Spirodipyranform überführt. Man erhält 13 Teile des Farbbildners 3'-Methyl-7-N-morpholinyl-spiro-(2H-1-benzopyran)-2,2'-(2H)-naphtho(2,1-b)-pyran der Formel

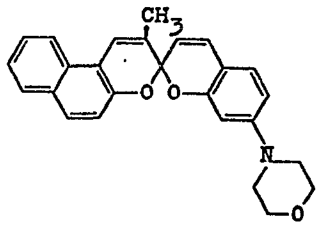

mit einem Schmelzpunkt von 154 bis 155°C.

In Kontakt mit sauer reagirenden Substanzen entwickelt die Verbindung eine Blaufärbung.

Praktisch keine Farbentwicklung erfolgt hingegen auf nicht mit Elektronenakzeptoren beschichtetem Papier. Eine vergleichsweise intensive blaugrüne Farbentwicklung erhält man mit einem Farbbildner, der durch eine Diäthylaminogruppe anstelle des Morpholinringes in 7-Stellung substituiert ist.

Beispiel 9

Entsprechend den Angaben in Beispiel 8 werden 21 Teile 2-Methyl-3-i-butyl-naphthopyryliumtrichlorozinkat und 11 Teile 4-N-Morpholinylsalicylaldehyd in 300 Teilen Alkohol zwei Stunden unter Rückfluß erhitzt.

Man erhält 5 Teile 3'-i-Butyl-7-N-morpholinyl-spiro(2H-1-benzopyran)-2,2'-(2H)-naphtho-(2,1-b)-pyran der Formel

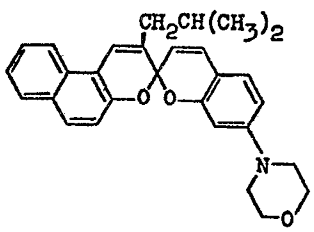

Der Farbbildner schmilzt bei 101 bis 102°C und entwickelt mit Elektronenakzeptorsubstanzen eine blaue Färbung.

Beispiel 10 und 11

Analog den Angaben in den Beispielen 8 und 9 werden die Farbbildner der Formel

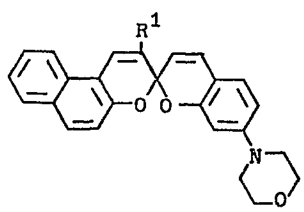

erhalten

Die Bedeutung von $R^1$ und die beim Kontakt mit sauer reagierenden Substanzen entwickelten Farbtöne sind in der folgenden Tabelle angegeben.

| Nr. | $R^1$ | Farbton |
|---|---|---|
| 10 | $i\text{-}C_5H_{11}$ | blau |
| 11 | $i\text{-}C_3H_7$ | blau |

**Patentansprüche**

1. Spirodipyrane der allgemeinen Formel

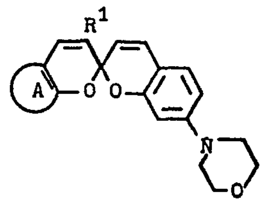

in der $R^1$ $C_1$- bis $C_{16}$-Alkyl und A den Rest eines ankondensierten Benzol- oder 2,1-Naphthalinringes bedeutet.

2. Spirodipyrane gemäß Anspruch 1, *dadurch gekennzeichnet,* daß in der Formel $R^1$ für $C_1$- bis $C_4$-Alkyl steht.

3. Verwendung der Spirodipyrane gemäß Anspruch 1 oder 2 als Farbbildner für druckempfindliche Aufzeichnungsmaterialien.

4. Druckempfindliches Aufzeichnungsmaterial, *dadurch gekennzeichnet,* daß dieses als Farbbildner Spirodipyrane gemäß Anspruch 1 oder 2 enthält.

**Revendications**

1. Spirodipyrannes de formule générale

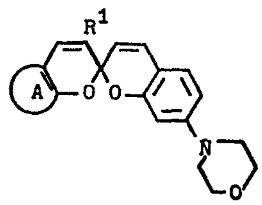

dans laquelle $R^1$ représente un alcoyle à 1—16 atomes de carbone et A le radical d'un noyau benzénique ou 2,1-naphtalénique fixé par condensation.

2. Spirodipyrannes selon la revendication 1, caractérisés en ce que, dans la formule, $R^1$ est mis pour un alcoyle à 1—4 atomes de carbone.

3. Utilisation des spirodipyrannes selon la revendication 1 ou 2 comme chromogène pour des matériels graphiques sensibles à l'effet de la pression.

4. Matériels graphiques sensibles à l'effet de la pression, caractérisés en ce qu'ils contiennent, en tant que chromogènes, des spirodipyrannes selon la revendication 1 ou 2.

**Claims**

1. Spirodipyrans of the general formula

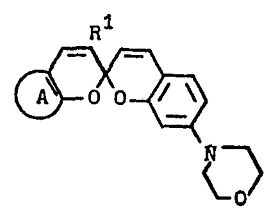

where $R^1$ is $C_1$- $C_{16}$-alkyl and A is the radical of a fused benzene or 2,1-naphthalene ring.

2. Spirodipyrans as claimed in claim 1, *characterised in that* $R^1$ in the formula is $C_1$-$C_4$-alkyl.

3. The use of the spirodipyrans as claimed in claim 1 or 2 as dye forming component for pressure-sensitive recording materials.

4. Pressure-sensitive recording materials, *characterised in that* they contain spirodipyrans as claimed in claim 1 or 2 as dye forming components.